# EUROPEAN PATENT SPECIFICATION

(11) **EP 2 319 428 B1**
(45) Date of publication and mention of the grant of the patent: **10.12.2014**
(21) Application number: 10180078.7
(22) Date of filing: 05.03.2001
(51) Int. Cl.: A61B 17/10

(54) **Suture clip**
Nahtklammer
Elément de blocage de suture

(30) Priority: 03.03.2000 US 186926 P; 19.05.2000 US 205741 P; 19.05.2000 US 205444 P; 29.11.2000 US 253970 P
(43) Date of publication of application: 11.05.2011
(62) Divisional of application: 01918427.4
(73) Proprietor: C. R. BARD, INC., Murray Hill, NJ 07974 (US)
(72) Inventor: Gambale, Richard A., Tyngsboro, MA 01879 (US); Weiser, Michael E., Groton, MA 01450 (US); Page, Edward Carlton, 01436, MA Baldwinville (US)
(74) Representative: Grey, Ian Michael

(56) References cited:
- WO-A1-95/32670
- WO-A1-97/30639
- WO-A1-99/04699
- US-A- 5 584 835
- US-A- 5 626 590

## Description

The disclosed invention relates generally to devices used to secure sutures. More particularly, the invention relates to suture clips used in conjunction with sewing devices used in flexible endoscopy; though it is also applicable to devices used in rigid endoscopy.

It is estimated that as many as 15,000,000 individuals in the United States suffer from stomach acid reflux into the lower esophageal region, commonly referred to as GERD (gastroesophageal reflux disorder). Although the illness may result from a wide variety of causes, it is ultimately the failure of the cardiac sphincter located above the stomach that enables a reflux event to occur. A surgical method developed to reduce reflux episodes involves forming tissue folds in the walls of the stomach to reduce the cross-sectional area of the gastroesophageal juncture to mimic the function of the cardiac sphincter. To perform these types of procedures, sewing devices used to suture the stomach wall into folds are used. The procedure typically involves a fiber optic endoscope introduced into the lower esophageal area. A sewing instrument is advanced down the working channel of the endoscope that has an aspiration port for generating negative pressure to suction stomach wall tissue into the sewing instrument where one or more sutures are implanted to hold the suctioned tissue in a folded condition known as a plication.

Sewing devices for this procedure are described in, for example, GB-A-2165559 and US Patent No. 5080663. According to these references, a sewing device is used for passing a thread through a tissue fold. The sewing device comprises a hollow needle movable between a first pre-tissue penetration position and a second position in which it passes through the tissue, with a thread carrier adapted to be attached to the thread and being receivable within the hollow needle.

Preferably, the sewing device comprises a body that defines a cavity within which the substrate portion can be held, for example, by means of suction. The hollow needle is mounted for movement in the body between the first and second positions. In some versions of the procedure, a suture is inserted into the tissue with a needle, the two ends of which are fed back out of the patient. Typically, a physician fashioned a series of half hitches to secure the suture to the subject tissue.

In other embodiments f the procedure, a tag attached to a distal end of the suture and contained within a lumen of the needle is inserted into and past the tissue. With the needle in an advanced position, i.e. with the needle distal tip extending distally beyond the pierced tissue, a pusher forces the tag out of the needle. After retraction of the needle from the tissue, the suture is retracted so that the tag contacts the tissue. The tag functions as an anchor that enables the suture to be secured to the tissue from the proximal end and also disperses the force applied to the tissue by the suture to prevent tearing of the tissue. Such a device and procedure is described in US Patent No. 5080663 to Mills et al.

One of the significant problems associated with these procedures is the time and number of intubations needed to perform the various procedures endoscopically. Due to a number of concerns, a patient is typically anesthetized for no more that approximately 40 minutes. In this period of time, procedures such as the GERD procedure must be performed to completion. In the GERD procedure, several intubations are performed to create several plications. As many as nine intubations are required to create just one plication. This is the case when half hitches are used to secure the suture. Each half hitch requires the hitch to be made outside the endoscope and then advanced down the endoscope with a pusher. Typically, six half hitches are used per suture thus six intubations are needed to secure the half hitches. The time needed for each intubation substantially reduces the working time to complete a GERD procedure.

One approach to solving this problem is disclosed in US Patent NO. 5584861 to Swain. The Swain patent discloses a suture clip and suture clip delivery device that is used in place of half hitch knots. The disclosed suture clip is a cylinder with a plug that can be releasably secured in the cylinder. The disclosed suture clip delivery device includes a tube, the distal end of which has a recess for receiving the suture clip. An axially movable stirrup is provided at the distal end that has the capacity to be moved from a first position that secures the suture clip to the tube and a second position that allows for the suture clip to be removed from the recess.

An aperture is provided in the cylinder to receive the suture. The cylinder is advanced over the suture that exits from a proximal end of the cylinder and enters the tube. An aperture in a sidewall of the tube provides egress for the suture. The plug is then advanced down the tube and into the cylinder. The interfacing walls of the cylinder and plug capture the suture. A pusher is used to force the plug into the cylinder while the stirrup maintains the suture clip in the recess. Following plug insertion, the stirrup, which is offset from the centre axis of the tube, is advanced distally from the distal end of the tube to release the suture clip from the tube.

Although the Swain device solves the problem of multiple half hitches, the overall design of the device has certain drawbacks. First, to successfully join the cylinder to the plug to form the suture clip, the stirrup must be physically maintained in a retracted position while an opposing force is applied with a pusher to the plug. Second, the presence of the stirrup inevitably prevents the tube and therefore, the suture clip from being placed tight against the sutured tissue. This opens the possibility for slack to develop between the clip and the tissue. This opens the possibility for slack to develop between the clip and the tissue, which can potentially lead to a relaxation of the desired tissue fold.

Suture anchors or clips and the means to deliver and secure them are quite common in the medical industry as they play a significant role in simplifying the tedious task of securing tissue previously accomplished by tying knots on sutures. Quite common are metallic twist tie, staples and various forms of plastic or metallic permanent or temporary mechanical means to prevent the suture from slipping through the tissue. As a result of the function, the clips are typically designed to be large to overcome the stresses expected of them. Disclosed are several single and multi-component suture clips as well as a variety of relatively simple compact suture clip delivery devices that can be inserted into a natural body orifice or through the working channel of an endoscope to cinch a suture clip into the desired position in close proximity to or against the plication.

It is to be appreciated that the suture clips disclosed herein have a potentially wide range of applications including, but not limited to, the attachment of devices, e.g. pH monitor to the gastrointestinal wall, the closure of perforations or ulcers and the creation of anastomoses. Another useful application involves the use of radiopaque clips as fluoroscopic markers.

WO-A-95/32670 discloses a non-locking suture clip according to the preamble to claim 1.

A non-locking suture clip according to the present invention is characterised by the features recited in the characterising portion of claim 1.

The suture clips described herein are designed to allow the suture to inter-wind through the clips in such a manner that the clips move with minimal friction while in an open position. In a closed position, the clip captures the suture, by the increased friction. The suture passes proximally through the chamber between the pusher head and mating tang and then outward through a lumen in the chamber and continues proximally outside the endoscope or catheter to the proximal end of the entire system. The use may thread a suture through a clip and then load the clip into the tooling. The clip may, in certain designs, already be premounted and then require the final advance by the physician to the site. At the site the physician activates the handle to apply a force to the clip, thereby locking it to the suture. The application of force first secures the clip and captures the suture material within the mating surfaces, and then expels the clip from the tool to remain within the patient.

Preferable features of the non-locking suture clip according to the invention are defined in the dependent claims numbers 2 to 15.

### Brief description of the drawings

Figure 1 is a side partial cutaway sectional view of an endoscope with an attached suture clip delivery/locking device and, a suture clip according to one embodiment of the invention in a first position;
Figure 1a is a perspective view of a suture clip delivery/locking device and, a suture clip according to another embodiment of the invention in a first position;
Figure 1b is a perspective view of a suture clip delivery/locking device and, suture clip according to another embodiment of the invention in a first position;
Figure 1c is a perspective view of a suture clip delivery/loclung device and, suture clip according to another embodiment of the invention in an advanced intermediate position;
Figure 1d is a perspective view of a suture clip delivery/locking device and suture clip according to another embodiment of the invention in a second open position;
Figure 2a is side elevational view of a catheter with an attached suture clip delivery device and, a suture clip according to one embodiment of the invention;
Figure 2b is a perspective view of a suture clip delivery/locking device and suture clip components in a pre-cinched state according to one embodiment of the invention;
Figure 2c is a partial sectional view of a suture clip delivery/locking device, and suture clip components in a pre-cinched state according to one embodiment of the invention;
Figure 2d is a partial sectional view of a suture clip delivery/locking device and, suture clip components in a partially cinched state according to one embodiment of the invention;
Figure 2e is a partial sectional view of a suture clip delivery/locking device and suture clip components in a partially cinched state, delivered according to one embodiment of the invention;
Figure 3 is a partial sectional view of a suture clip delivery/locking device and, suture clip components in a pre-cinched state according to one embodiment of the invention;
Figure 4 is a perspective view of a suture clip delivery/locking device and, suture clip according to one embodiment of the invention;
Fiugure 4a is a perspective view of a suture clip delivery/locking device and, a suture clip according to one embodiment of the invention;
Figure 5 is a side elevational view of a plication with a suture clip attached to a suture according to one embodiment of the invention;
Figure 6 is a perspective view of a suture clip assembly in an open position according to one embodiment of the invention;
Figure 7 is a perspective view of a suture clip assembly in a closed position according to one embodiment of the invention;
Figure 8 is a fragmented side view of a suture clip assembly according to one embodiment of the invention;
Figure 9 is a perspective view of a suture clip assembly in a partially closed position according to a further embodiment of the invention;
Figure 10 is a perspective view of a suture clip assembly in a partially closed position according to one embodiment of the invention;
Figure 11 is a side elevational view of a suture clip assembly in an open position according to another embodiment of the invention;
Figure 12 is a side elevational view of a suture clip assembly in a closed position according to one embodiment of the invention;
Figure 13 is a top view of a suture clip assembly according to one embodiment of the invention;
Figure 14 is a perspective view of a suture clip assembly according to a further embodiment of the invention;
Figure 15 is a side elevational view of a suture clip assembly in a closed position according to a further embodiment of the invention;
Figure 16 is a side elevational view of a suture clip assembly in a partially closed position according to another embodiment of the invention;
Figure 17 is a side sectional view of a single pusher suture clip locking and severing catheter distal end loaded according to one embodiment of the invention, with a convex head suture clip plug and suture clip ring'
Figure 18 is a top perspective view of an assembled suture lock plug and suture lock ring according to another embodiment of the invention;
Figure 19 is a sectional view of an assembled suture lock plug and suture lock ring according to a further embodiment of the invention;
Figure 20 is an end view of an assembled suture lock plug and suture lock ring according to a further embodiment of the invention;
Figure 21 is a perspective view of an unassembled suture lock plug and suture lock ring according to a further embodiment of the invention;
Figure 22 is a perspective view of a suture lock plug according to a yet further embodiment of the invention;

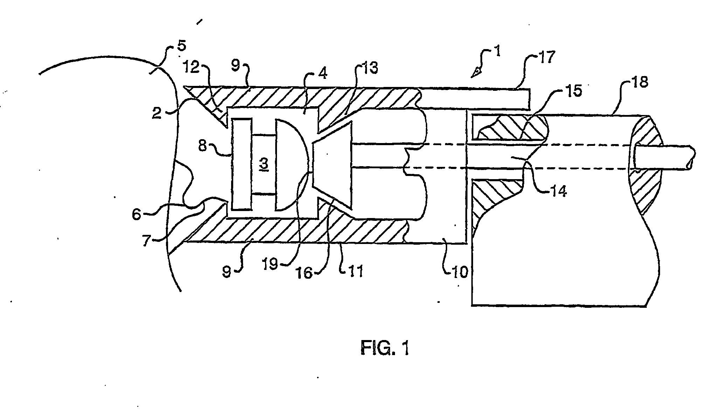

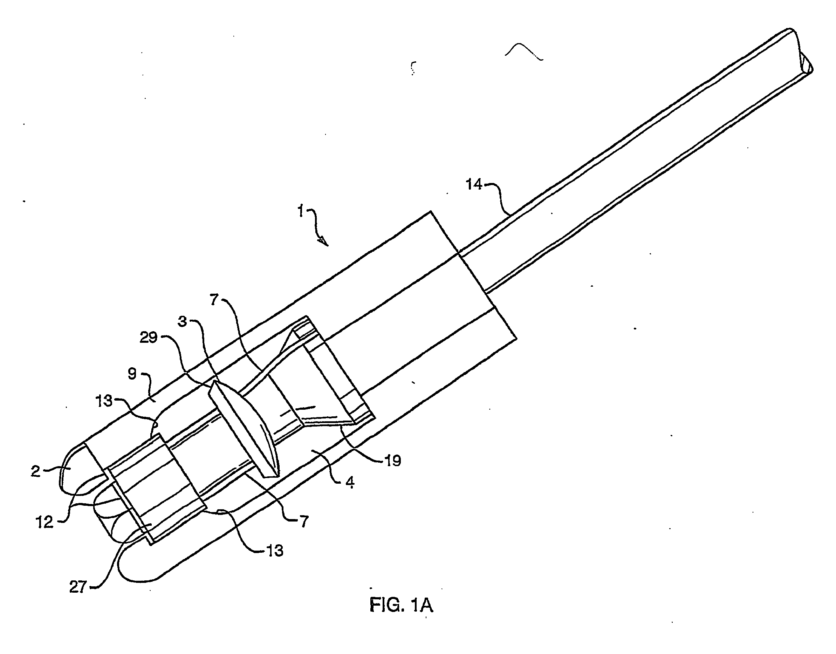

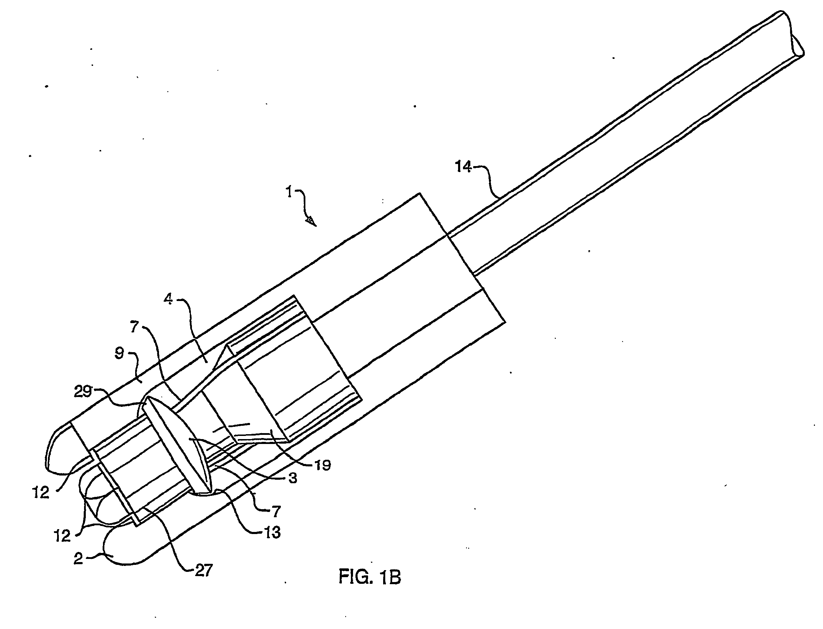

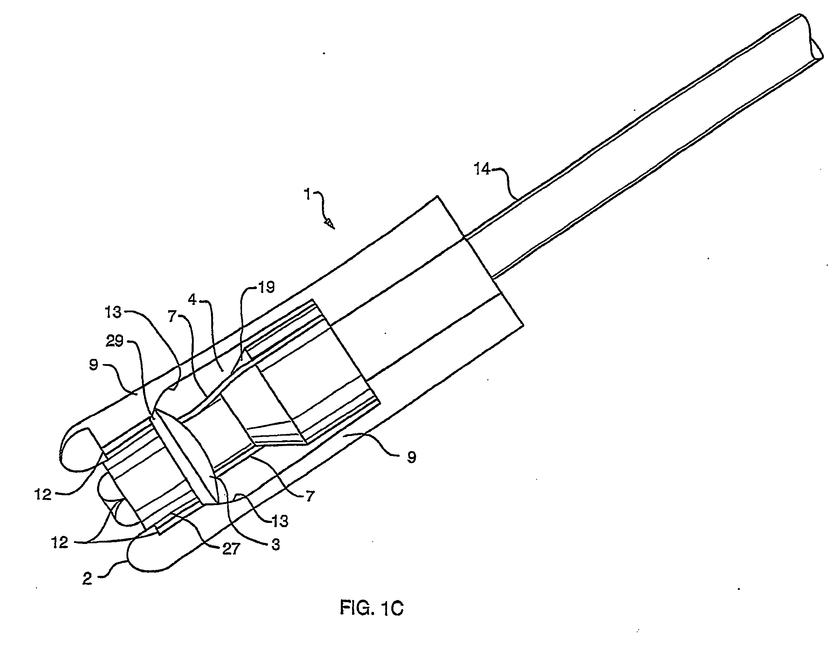

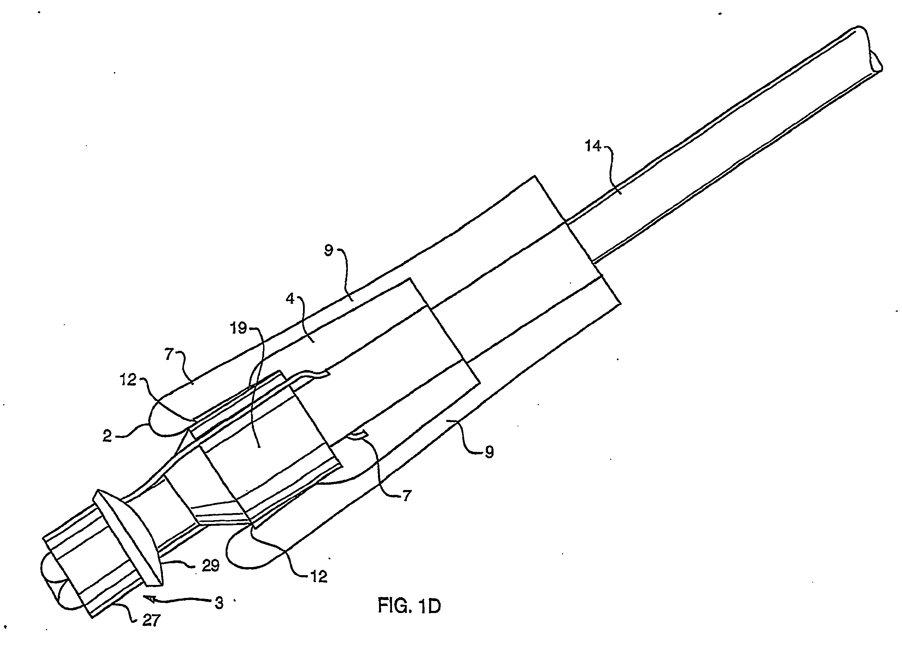

Referring to Figures 1-3, a clip delivery/locking device is shown generally as 1. Device 1 is shown in an embodiment designed for mounting the distal end of an endoscope 18. Device 1 is generally cylindrical in shape and has a flange 17 extending proximally from a proximal end of device 1. Flange 17 is adapted to conform to the contours of the exterior surface of endoscope 18. The combination of the proximal end of device 1 interfacing with the distal end of endoscope 18 and flange 17 interfacing with the exterior surface of endoscope 18 effectively locks device 1 to endoscope 18. Alternatively, device 1 can be secured to endoscope 18 with mechanical fasteners.

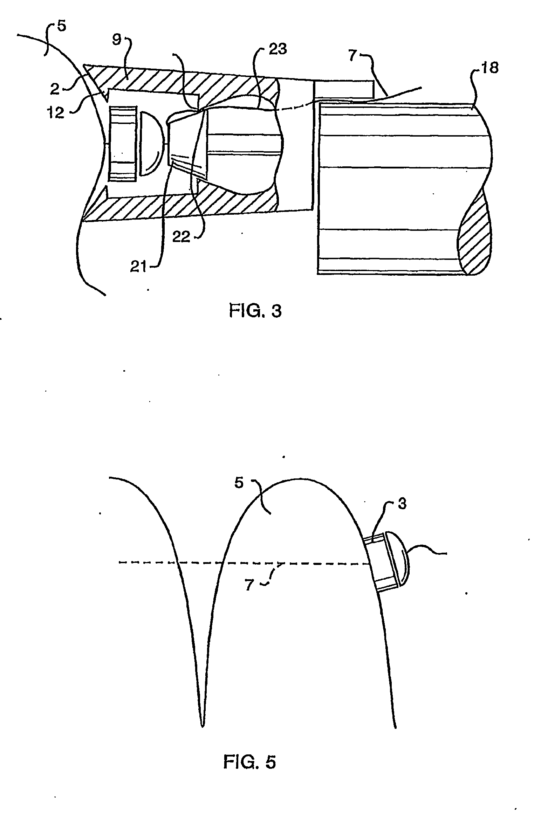

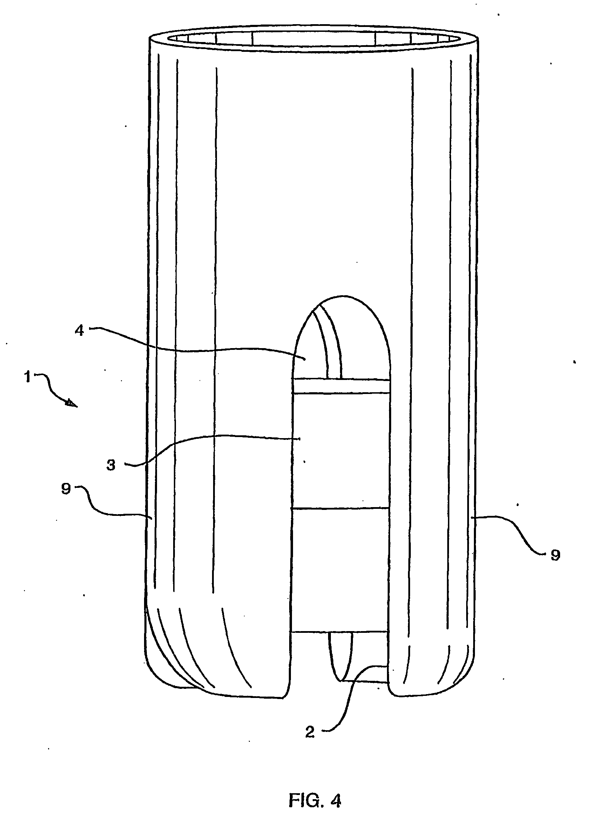

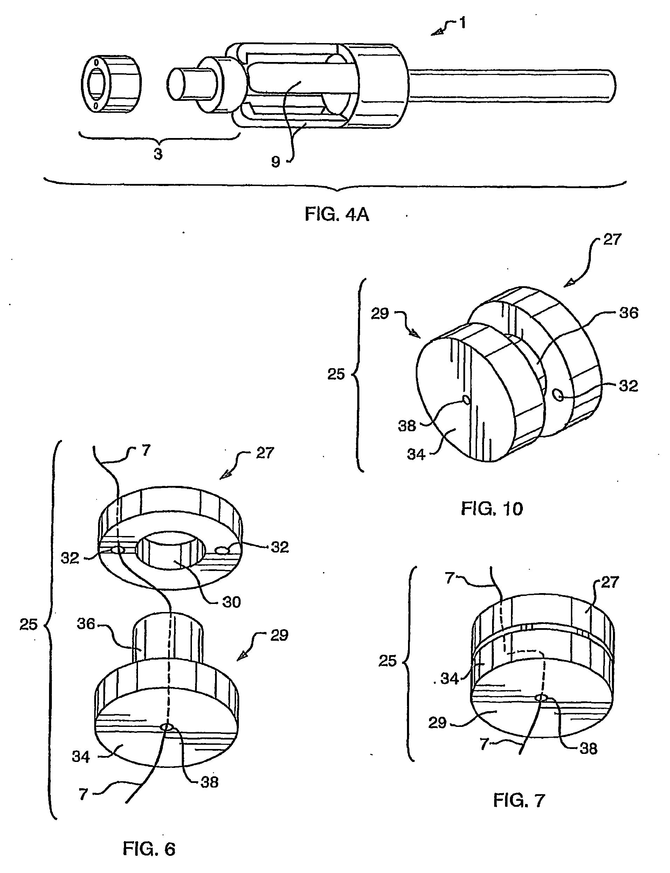

Device 1 has portions defining a clip holding chamber 4. A proximal end 4a of holding chamber 4 (shown in Figure 4) formed on the proximal end of device 1 communicates with holding chamber 4 and a working channel 15 of endoscope 18. Extending distally from a distal end of device 1 are fingers 9 that are oriented on opposite sides of, and partially define, holding chamber 4. Fingers 9 are designed to flex or spring from a first position (shown in Figure 1) to a second position (shown in Figure 3) to deliver a suture clip assembly comprising a plug 3 and a ring 8. After delivery, fingers 9 spring back to the first position to receive another suture clip assembly. Preferably four fingers 9 are provided (as shown in Figure 4a) although as little as two fingers 9 are needed. When viewing the distal end of device 1, the distal ends of fingers 9 preferably form a segmented 360° ring.

Fingers 9 have tapered finger ends 2 with the taper preferably increasing radially outwardly from a proximal end to a distal end of finger ends 2. The proximal ends of finger ends 2 extend radially inwardly of the inner walls of fingers 9 to form distal tangs 12. Distal tangs 12 provide a stop surface against which suture clip 3 can be compressed for manipulation as described in detail below and by which premature release of suture clip 3 is prevented.

Extending radially inwardly from fingers 9 from a point of distal tangs 12 are proximal tangs 13. Proximal tangs 13 are preferably tapered with the taper increasing radially inwardly from a proximal end to a distal end of proximal tangs 13. In an alternate embodiment shown in Figures 1a-1d, proximal tangs 13 are radiused portions of the inner walls of fingers 9 that merge into finger ends 2. As described in more detail below, proximal tangs 13 facilitate the movement of fingers 9 from the first position to the second position and act as a cam follower to distal tangs 12.

A pusher 14 is dimensioned and adapted to move freely in an axial direction with the working channel of endoscope 18 and within delivery device 1. Pusher 14 preferably has a frusto-conically shaped distal end 19, the tapered sides of which are dimensioned to contact proximal tangs 13 and translate distal axial movement into radial outward movement of fingers 9.

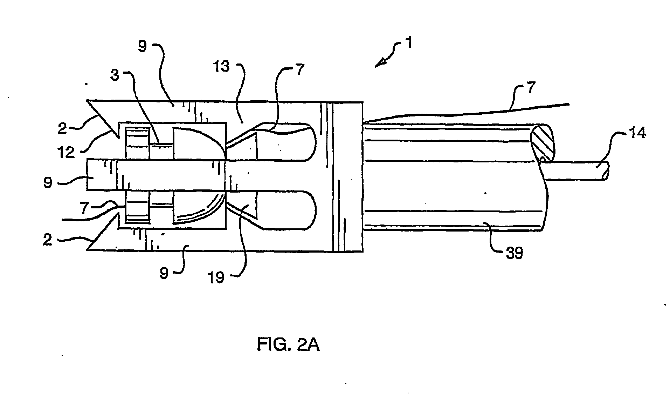

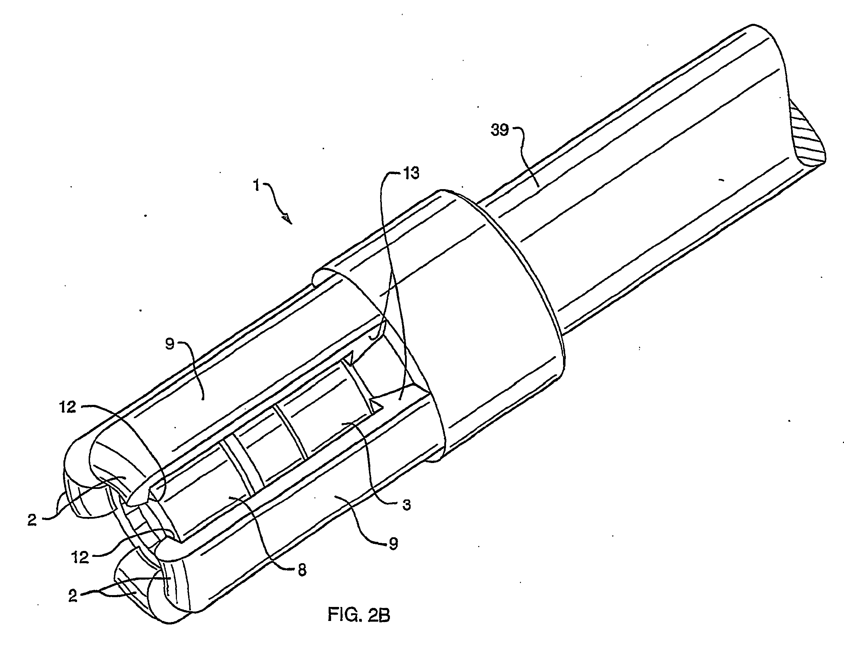

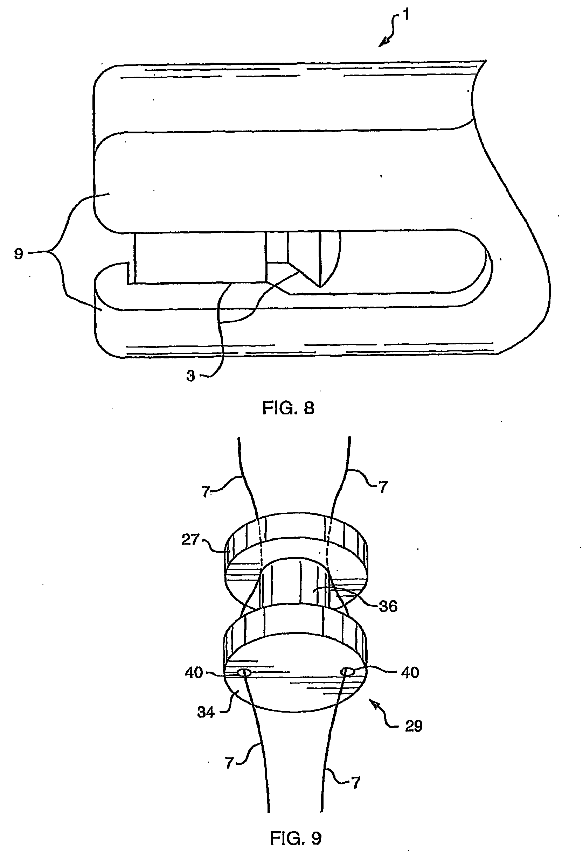

An alternative to the delivery device shown in Figures 1 and 2a is shown in Figures 2b-2e. This alternative can be used with an endoscope or independently as a catheter. In this embodiment, pusher 14 has a reduced diameter, substantially cylindrical distal end 91a. The transition from the diameter of a main body of pusher 14 and distal end 19 forms tapered pusher surface 19b dimensioned to contact proximal tangs 13 to affect the translation of distal axial motion of pusher 14 into radial outward motion of figures 9. Sutures 7 are severed at shoulder 9a formed at the transition of the main body of delivery device 1 and proximal tangs 13 when tapered pusher surface 19b contacts and slides along proximal tangs 13.

To operate device 1, a suture clip assembly 3a (plug 3 and ring 8), is advanced down endoscope working channel 15 in an open position with pusher 14. Depending on the particular embodiment of suture clip assembly 3a used, any sutures placed inside the subject patient are threaded into any of a variety of apertures in suture clip assembly 3a before suture clip assembly 3a descends down working channel 15.

With suture clip assembly 3a in an open position, sutures 7 ride through ring 8 and plug 3 without becoming locked. The specific procedure used for each embodiment of suture clip assembly 3a described herein will be provided below. Alternatively, the procedure can be initiated with suture clip assembly 3a being premounted in holding chamber 4 before intubation of endoscope 18.

Pusher 14 preferably runs axially along the entire axial length of endoscope 18 so that it can be manipulated from the proximal end of endoscope 18. For the embodiment of device 1 described above and illustrated in Figure 1, pusher 14 preferably has a frusto-conically shaped pusher distal end 19 the taper of which preferably conforms to the taper of proximal tangs 13. It is to be understood that pusher distal end 19 need not be given any particular shape. For example, a cylindrically shaped pusher distal end 19 can also be used provided that the cross-sectional diameter is sufficiently large to contact with proximal tangs 13 to spread fingers 9.

It is important to the function of device 1 that suture clip 3 have a sufficient diameter, if circular in cross-section, or width, if square or rectangular in cross-section, to contact proximal tangs 13. It is equally important that suture clip 3 has an axial length, in an open position that is no greater than the distance between the opposing surfaces of distal tangs 12 and proximal tangs 13.

Suture clip 3 is urged into device 1 until contact is made with proximal tangs 13. Force is then applied to pusher 14 to force suture clip 3 against proximal flanges 13. Sufficient force is applied so that fingers 9 spread radially outwardly as a result of the sliding contact of proximal tangs 13 with the outer surface of suture clip 3. Once suture clip 3 is advanced past proximal tangs 13, fingers 9 spring back into the first position. When in the first position, distal tangs 12 contact suture clip 3 and stop the advancement of suture clip 3.

To advance suture clip 3 past device 1, additional force is applied to pusher 14 so that pusher distal end 19 contacts and advances along proximal tangs 13 which causes finger 9 to flex radially outwardly into the second position. Pusher distal end 19 simultaneously pushes suture clip 3, now disengaged with distal tangs 12, out of the distal end of device 1 against a plication 5.

Unlike other prior art designs, the design of device 1 allows for direct contact between plication 5 and device 1 so that the placement of a suture clip can be compressed against the plication with minimal or no slack in the suture as shown in Figure 4. This ensures the suture will properly maintain the plication in the desired bound state.

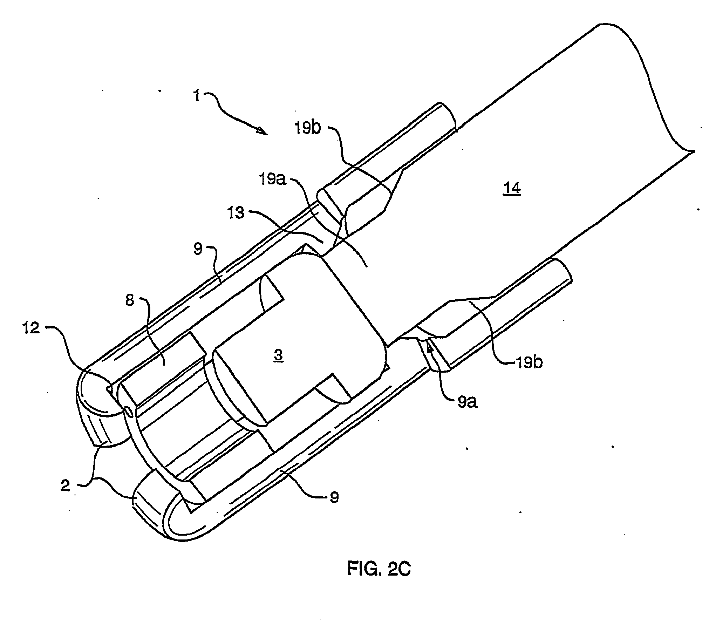

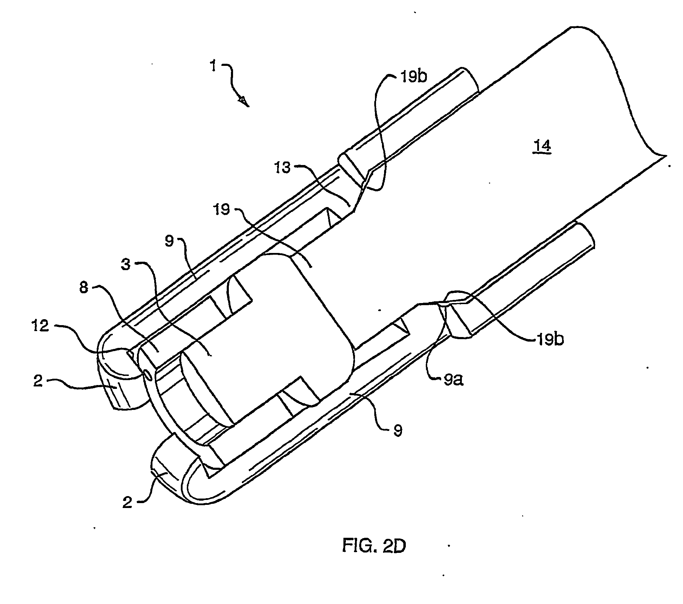

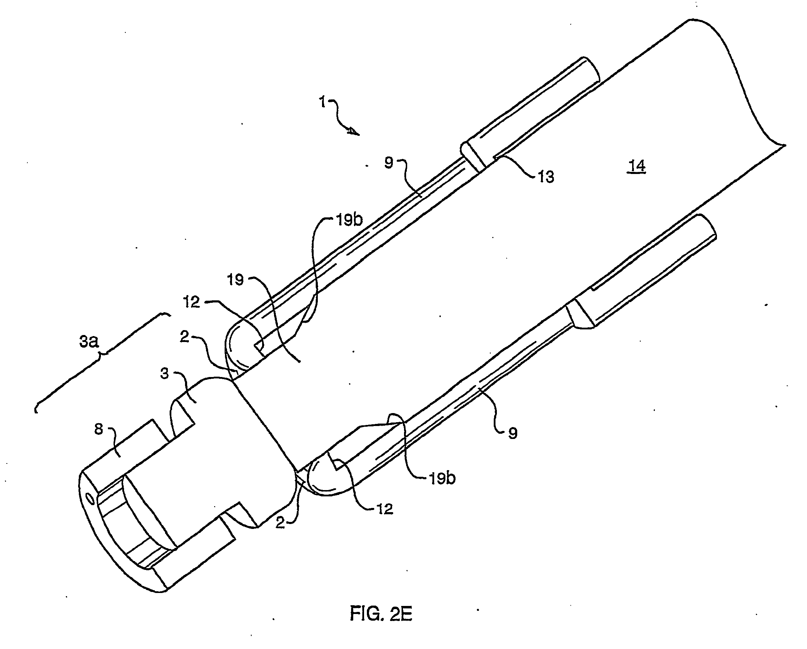

Referring to Figure 2, another device 1 is shown attached to a distal end of a catheter 20. The procedure described above with respect to an endoscope also applies to a catheter-based version of device 1 except that in this embodiment, pusher 14 is preferably run through a central lumen of catheter 20.

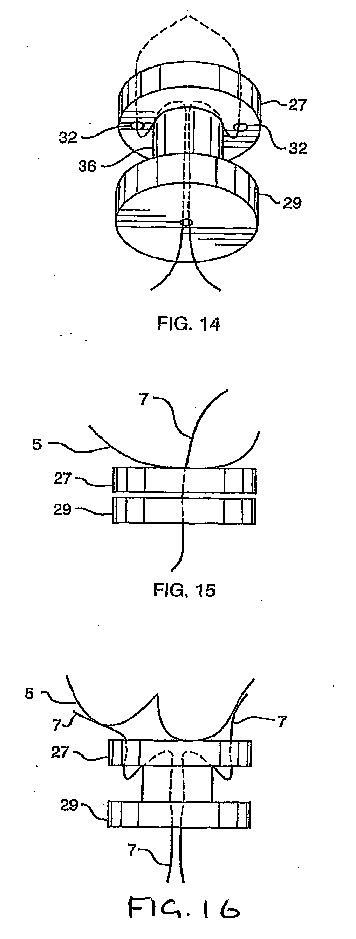

Referring to Figures 6-16, a variety of suture clip embodiments are shown that employ a friction fit means to secure one or more sutures. As shown in Figures 6,7 and 10, a suture clip assembly 25 is shown comprised of a suture disk 27 and a disk post 29. In this embodiment, suture disk 27 has portions defining a central disk aperture 30 that is preferably located in the radial center of suture disk 27. Suture apertures 32 are formed in suture disk 27 radially outwardly from disk aperture 30. Suture apertures 32 are adapted to receive sutures 7. One or more suture apertures 32 can be formed in suture disk 27.

Disk post 29 has a disk post main body 34 that is preferably circular with a diameter that matches the diameter of suture disk 27. A locking post 36 extends axially from a face of disk post 29 and is preferably an integral part of disk post 29. Locking post 36 has a cross-sectional diameter that is sized to fit tightly within disk aperture 30. A post aperture 38 runs axially through disk post 29 and locking post 36. Post aperture 38 is adapted to receive sutures 7 and is preferably located in the radial center of disk post 29.

In the embodiment shown in Figure 6, locking post 36 has a smooth outer surface. As shown in Figure 8, locking post 36 has a corrugated surface that can also be formed with threading to enhance grasping of sutures 7 when suture clip assembly 25 is manipulated into a closed position. A shown in Figure 8a, locking post 36 and disk aperture 30 can be formed with matching tapers that allow for one-way insertion of locking post 36 into disk aperture 30 to further enhance grasping of sutures 7.

To use this embodiment of suture clip assembly 25, a suture 7 (extending out of the mouth of the patient in an endoscopic procedure), is thread through post aperture 38 from a distal end of disk post 34 to a proximal face of disk post main body 34. Suture 7 is then fed through suture aperture 32 from a distal face of suture disk 27 to a proximal face of suture disk 27. Both suture disk 27 and disk post 29 are slid along suture 7 until in place in holding chamber 4 of device 1.

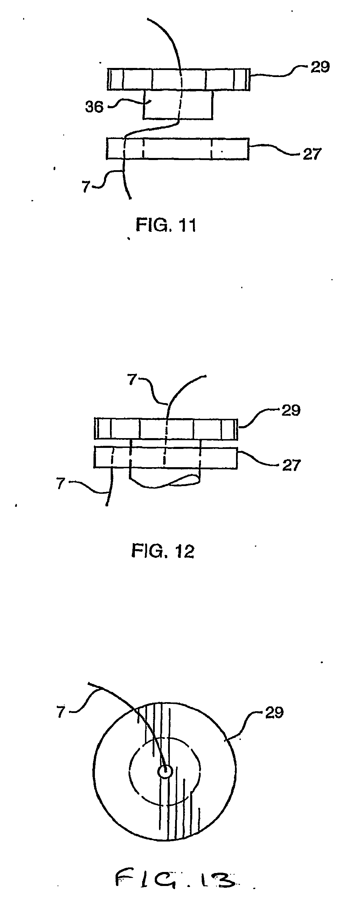

Before securing suture disk 27 to disk post 29, any slack in suture 7 is taken up by pulling suture 7 in a proximal direction. To engage disk post 29 to suture disk 27, Force is applied to pusher 14 which forces locking post 36 into disk central aperture 30. Suture 7 becomes entrapped or captured between the mating surface of locking post 36 and disk central aperture 30 as well as between the proximal face of suture disk 27 and the distal face of disk post 29. The tortuous path followed by suture 7 adds to the friction achieved between suture 7 and suture clip assembly 25. Figures 11-16 show the path followed by one suture 7 and dual sutures 7 in this embodiment.

Once suture clip assembly 25 has been assembled, suture 7 is captured with suture clip assembly 25 interfacing with plication 5. Suture 7 passes between proximal tang 13 and pusher distal end 19 such that when distal end 19 is forced against proximal tang 13, suture 7 captured. An edge 23 formed in device 1 acts as a knife to sever suture 7 when suture 7 is tensioned by pulling suture 7 proximally while pusher 14 is forced distally through device 1.

In an alternative embodiment shown in Figure 9, suture disk 27 is not formed with suture apertures 32. Instead, disk post main body 34 is provided with disk post suture apertures 40 that are formed radially outwardly from locking post 36. Sutures 7 are fed through central aperture 30 and through disk post suture apertures 40. Joining of suture disk 27 and disk post 29 results in the same capture of sutures 7 as previously described.

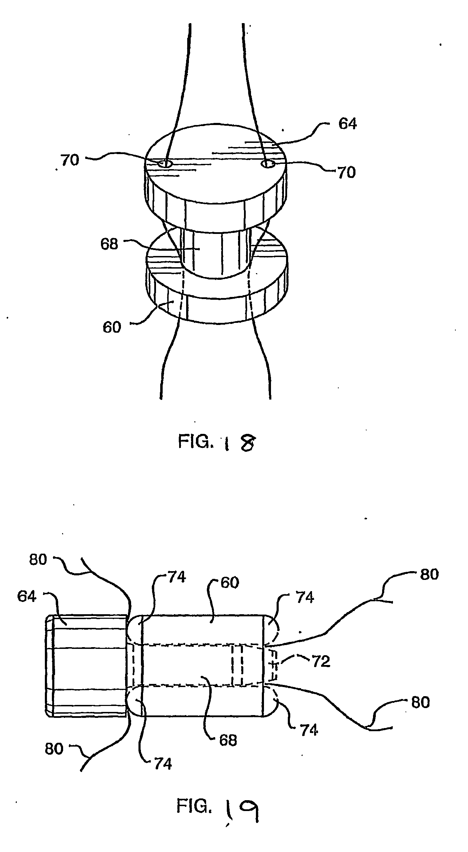

The suture clips used with the suture clip delivery catheter and/or endoscopic system described herein, have plugs with heads. In another embodiment as shown in Figure 18, plug 62 has distal shaft 68 that is adapted to frictionally engage the inner walls of ring 60 and a head 64 that has preferably two suture receiving bores 70 formed toward the perimeter of the head 64. This configuration provides a suture clip plug that decreases the effort needed to advance the plug over the ends of a suture before loading into the suture clip delivery device.

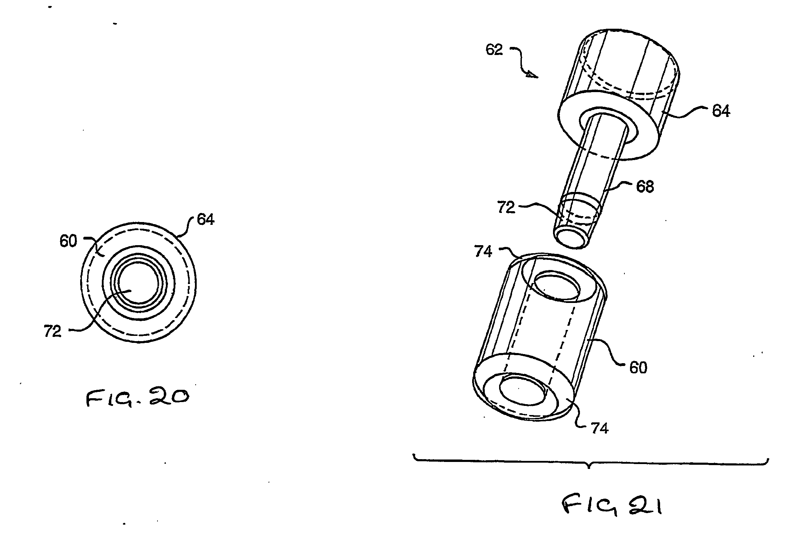

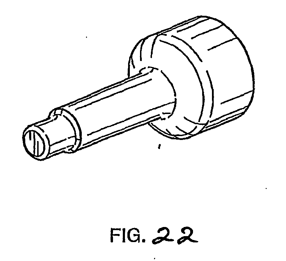

In a preferred embodiment shown in Figures 19-21, the inner and outer walls of ring 60 are radiused at the ends 74 to facilitate the insertion of distal shaft 68 into ring 60. More importantly, the radiused ends provide strain relief for the sutures 80 when compressed between the plug 62 and ring 60. To further facilitate insertion, distal shaft 68 is formed with a tapered distal tip 72. Alternatively, a reduced neck distal tip 72a can be employed as shown in Figure 22. Although interlocking features can be incorporated into the plug and clip components, it has been found that such features can lead to suture fraying, premature fracturing and failure. Use of tapered or reduced tipped plugs 62 allow for a preferred gradual reduction in compression. It has been found that suture capture is optimized and suture destruction is minimized when a distally decreasing taper leading end is used on plug 62 with a proximally increasing diameter of plug 62 to exceed the net diameter of the suture 80 in a captured state plus the diameter of plug 62 by at least 0.051mm (.002 inches). The preferred materials used to make plug 62 and ring 60 are polyetherether ketone (PEEK) 450G, PEEK-Optima™ LT or polyethylene terephthalate (PET).

As previously stated, suture 80 is held via friction between plug 62 and ring 60. For example, a 0.229mm (.009 inch) thick suture is captured in a 0.0762mm (.003 inch) gap between plug 62 and ring 60 so that the suture will remain captured with the application of a three pound load. The aforementioned radiused and tapered surfaces of plug 62 and ring 60 provide enough strain relief to prevent damage to suture 80 when subjected to the three-pound load.

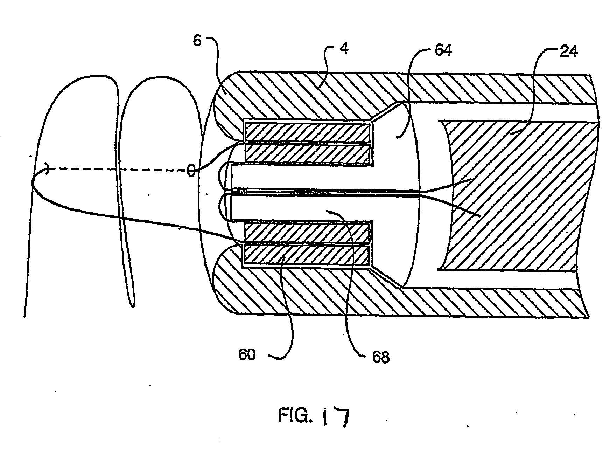

A further suture clip embodiment employs a head with a proximal surface that is convex as shown in Figure 17. Preferably, the distal end of inner pusher 24 is provided with a concave surface to matingly engage the convex surface of plug head. This configuration aids with the axial alignment of the ring and plug during distal advancement and engagement.

It should be understood that the foregoing description of the invention is intended merely to be illustrative thereof.

## Claims

1. A non-locking suture clip comprising:
a plug (3, 29, 62) having a head (34,64) and a shaft (36,68) extending distally from the head (34,64), the shaft (36,68) having an outer surface defining a first diameter and the head (34,64) having a second diameter greater than the first diameter; and
a ring (8,60)having an inner annular wall defining a through aperture (30) extending from a proximal end to a distal end of the ring (8,60), the through aperture (30) having an aperture diameter greater than the first diameter wherein insertion of the shaft (36,68) into the through aperture (30) forms an axial vacancy between the outer surface of the shaft (36,68) and the annular wall that is adapted to receive a suture therein such that it is held via friction between the outer surface of the shaft (36,68) and the annular wall, the plug (3,29) and the ring (8,60) having a non-locking arrangement wherein the plug (3,29) and the ring (8,60) do not lock together when the shaft (36,68) is inserted into the through aperture (30) without the suture located in the axial vacancy, **characterised in that** the shaft (36,68) and ring (8,60) are configured to hold a suture between the outer surface of the shaft (36,68) and the annular wall of the ring (8,60) for the length of the axial vacancy.

2. The non-locking suture clip of claim 1, wherein the shaft (68) has a tapered distal end (72).

3. The non-locking suture clip of claim 2, wherein the tapered distal end (72) tapers inwardly in a direction extending away from the head (64).

4. The non-locking suture clip of claim 1, wherein the shaft (68) has a reduced neck distal end (72a).

5. The non-locking suture clip of any of claims 1 to 4, wherein the axial vacancy is at least about 0.076mm (0.003 inches).

6. The non-locking suture clip of any of claims 1 to 4, wherein the axial vacancy is no greater than about 0.076mm (0.003 inches).

7. The non-locking suture clip of any of claims 1 to 6, further comprising a suture (7) situated between the plug shaft (36,68) and the ring (8,60) wherein the suture has a diameter greater than the axial vacancy.

8. The non-locking suture clip of any of claims 1 to 7, wherein the aperture diameter of the through aperture (30) is less than the second diameter so that the head (34,64) cannot be inserted into the through aperture (30).

9. The non-locking suture clip of any of claims 1 to 8, wherein the plug (3,29) and the ring (8,60) are adapted to be implanted within a human body.

10. The non-locking suture clip of any of claims 1 to 9, wherein the ring (8,60) is continuous.

11. The non-locking suture clip of any of claims 1 to 10, wherein the ring (8,60) comprises one or more ring suture apertures (32) extending through the ring (8,60) from the proximal end to the distal end of the ring (8,60), each ring suture aperture (32) being configured to receive a suture.

12. The non-locking suture clip of claim 7, wherein the ring (8,60) comprises two ring suture apertures (32) positioned oppositely from each other.

13. The non-locking suture clip of any of claims 1 to 12, wherein the plug (3,29) has one or more plug suture apertures (38,40,70) extending through the plug from the proximal end to the distal end of the plug (3,29), each plug suture aperture (40,70) configured to receive a suture.

14. The non-locking suture clip of claim 13, wherein the plug (3,29) has a single plug suture aperture (38) located in the radial centre of the plug (3,29).

15. The non-locking suture clip of claim 13, wherein the plug (3,29) has two plug suture apertures (40,70), located oppositely from each other and near the radial edge of the head (34,64) of the plug (3,29,62).

## Patentansprüche

1. Nicht arretierende Nahtklammer, die Folgendes umfasst:
einen Stopfen (3, 29, 62) mit einem Kopf (34, 64) und einem Schaft (36, 68), der sich distal von dem Kopf (34, 64) erstreckt, wobei der Schaft (36, 68) eine Außenfläche aufweist, die einen ersten Durchmesser definiert, und der Kopf (34, 64) einen zweiten Durchmesser hat, der größer ist als der erste Durchmesser; und
einen Ring (8, 60) mit einer ringförmigen Innenwand, die eine Durchgangsöffnung (30) definiert, die von einem proximalen Ende zu einem distalen Ende des Rings (8, 60) verläuft, wobei die Durchgangsöffnung (30) einen Öffnungsdurchmesser hat, der größer ist als der erste Durchmesser, wobei beim Einführen des Schafts (36, 68) in die Durchgangsöffnung (30) ein axialer Freiraum zwischen der Außenfläche des Schafts (36, 68) und der ringförmigen Wand entsteht, der einen Faden so darin aufnehmen kann, dass er durch Reibung zwischen der Außenfläche des Schafts (36, 68) und der ringförmigen Wand gehalten wird, wobei der Stopfen (3, 29) und der Ring (8, 60) eine nicht arretierende Anordnung haben, wobei der Stopfen (3, 29) und der Ring (8, 60) nicht aneinander arretiert werden, wenn der Schaft (36, 68) in die Durchgangsöffnung (30) gesteckt wird, ohne dass sich der Faden in dem axialen Freiraum befindet, **dadurch gekennzeichnet, dass** der Schaft (36, 68) und der Ring (8, 60) zum Halten eines Fadens zwischen der Außenfläche des Schafts (36, 68) und der ringförmigen Wand des Rings (8, 60) über die Länge des axialen Freiraums konfiguriert sind.

2. Nicht arretierende Nahtklammer nach Anspruch 1, wobei der Schaft (68) ein konisch verjüngtes distales Ende (72) hat.

3. Nicht arretierende Nahtklammer nach Anspruch 2, wobei das konisch verjüngte distale Ende (72) einwärts in einer vom Kopf (64) weg verlaufenden Richtung konisch verjüngt ist.

4. Nicht arretierende Nahtklammer nach Anspruch 1, wobei der Schaft (68) ein distales Ende (72a) mit reduziertem Hals hat.

5. Nicht arretierende Nahtklammer nach einem der Ansprüche 1 bis 4, wobei der axiale Freiraum wenigstens etwa 0,076 mm (0,003 Zoll) beträgt.

6. Nicht arretierende Nahtklammer nach einem der Ansprüche 1 bis 4, wobei der axiale Freiraum maximal etwa 0,076 mm (0,003 Zoll) beträgt.

7. Nicht arretierende Nahtklammer nach einem der Ansprüche 1 bis 6, die ferner einen Faden (7) umfasst, der sich zwischen dem Stopfenschaft (36, 68) und dem Ring (8, 60) befindet, wobei der Faden einen Durchmesser hat, der größer ist als der axiale Freiraum.

8. Nicht arretierende Nahtklammer nach einem der Ansprüche 1 bis 7, wobei der Öffnungsdurchmesser der Durchgangsöffnung (30) geringer ist als der zweite Durchmesser, so dass der Kopf (34, 64) nicht in die Durchgangsöffnung (30) gesteckt werden kann.

9. Nicht arretierende Nahtklammer nach einem der Ansprüche 1 bis 8, wobei der Stopfen (3, 29) und der Ring (8, 60) zum Implantieren in einen menschlichen Körper ausgelegt sind.

10. Nicht arretierende Nahtklammer nach einem der Ansprüche 1 bis 9, wobei der Ring (8, 60) fortlaufend ist.

11. Nicht arretierende Nahtklammer nach einem der Ansprüche 1 bis 10, wobei der Ring (8, 60) eine oder mehrere Ringfadenöffnungen (32) aufweist, die vom proximalen Ende zum distalen Ende des Rings (8, 60) durch diesen verlaufen, wobei jede Ringfadenöffnung (32) zum Aufnehmen eines Fadens konfiguriert ist.

12. Nicht arretierende Nahtklammer nach Anspruch 7, wobei der Ring (8, 60) zwei Ringfadenöffnungen (32) umfasst, die einander gegenüber liegend positioniert sind.

13. Nicht arretierende Nahtklammer nach einem der Ansprüche 1 bis 12, wobei der Stopfen (3, 29) eine oder mehrere Stopfenfadenöffnungen (38, 40, 70) umfasst, die vom proximalen Ende zum distalen Ende des Stopfens (3, 29) durch diesen verlaufen, wobei jede Stopfenfadenöffnung (40, 70) zum Aufnehmen eines Fadens konfiguriert ist.

14. Nicht arretierende Nahtklammer nach Anspruch 13, wobei der Stopfen (3, 29) eine einzelne Stopfenfadenöffnung (38) hat, die sich in der radialen Mitte des Stopfens (3, 29) befindet.

15. Nicht arretierende Nahtklammer nach Anspruch 13, wobei der Stopfen (3, 29) zwei Stopfenfadenöffnungen (40, 70) hat, die sich einander gegenüber liegend und in der Nähe des radialen Randes des Kopfes (34, 64) des Stopfens (3, 29, 62) befinden.

## Revendications

1. Élément de blocage de suture non verrouillable comprenant ;
un bouchon (3, 29, 62) ayant une tête (34, 64) et une tige (36, 68) s'étendant distalement à partir de la tête (34, 64), la tige (36, 68) ayant une surface externe définissant un premier diamètre et la tête (34, 64) ayant un second diamètre supérieur au premier diamètre ; et
un anneau (8, 60) ayant une paroi annulaire interne définissant une ouverture débouchante (30) s'étendant à partir d'une extrémité proximale vers une extrémité distale de l'anneau (8, 60), l'ouverture débouchante (30) ayant un diamètre d'ouverture supérieur au premier diamètre, dans lequel l'insertion de la tige (36, 68) dans l'ouverture débouchante (30) forme un vide axial entre la surface externe de la tige (36, 68) et la paroi annulaire, qui est conçu pour y recevoir une suture de façon à ce qu'elle soit maintenue par frottement entre la surface externe de la tige (36, 68) et la paroi annulaire, le bouchon (3, 29) et l'anneau (8, 60) ayant un agencement non verrouillable dans lequel le bouchon (3, 29) et l'anneau (8,60) ne se bloquent pas l'un dans l'autre lorsque la tige (36, 68) est introduite dans l'ouverture débouchante (30) sans que la suture soit placée dans le vide axial, **caractérisé en ce que** la tige (36, 68) et l'anneau (8, 60) sont agencés de façon à maintenir une suture entre la surface externe de la tige (36, 68) et la paroi annulaire de l'anneau (8, 60) sur la longueur du vide axial.

2. Élément de blocage de suture non verrouillable selon la revendication 1, dans laquelle la tige (68) a une extrémité distale effilée (72).

3. Élément de blocage de suture non verrouillable selon la revendication 2, dans laquelle l'extrémité distale effilée (72) diminue progressivement vers l'intérieur selon une direction s'étendant à l'opposé de la tête (64).

4. Élément de blocage de suture non verrouillable selon la revendication 1, dans laquelle la tige (68) a une extrémité distale de col réduite (72 a).

5. Élément de blocage de suture non verrouillable selon l'une quelconque des revendications 1 à 4, dans laquelle le vide axial mesure au moins environ 0,076 mm (0,003 pouces).

6. Élément de blocage de suture non verrouillable selon l'une quelconque des revendications 1 à 4, dans laquelle le vide axial est inférieur ou égal à environ 0,076 mm (0,003 pouces).

7. Élément de blocage de suture non verrouillable selon l'une quelconque des revendications 1 à 6, comprenant en outre une suture (7) placée entre la tige du bouchon (36, 68) et l'anneau (8, 60), dans laquelle la suture a un diamètre supérieur au vide axial.

8. Élément de blocage de suture non verrouillable selon l'une quelconque des revendications 1 à 7, dans laquelle le diamètre d'ouverture de l'ouverture débouchante (30) est inférieur au second diamètre de sorte que la tête (34, 64) ne puisse être introduite dans l'ouverture débouchante (30).

9. Élément de blocage de suture non verrouillable selon l'une quelconque des revendications 1 à 8, dans laquelle le bouchon (3, 29) et l'anneau (8, 60) sont conçus pour être implantés dans un organisme humain.

10. Élément de blocage de suture non verrouillable selon l'une quelconque des revendications 1 à 9, dans laquelle l'anneau (8, 60) est continu.

11. Élément de blocage de suture non verrouillable selon l'une quelconque des revendications 1 à 10, dans laquelle l'anneau (8, 60) comprend une ou plusieurs ouvertures d'anneau pour suture (32) s'étendant à travers l'anneau (8, 60) à partir de l'extrémité proximale vers l'extrémité distale de l'anneau (8, 60), chaque ouverture d'anneau pour suture (32) étant conçue pour recevoir une suture.

12. Élément de blocage de suture non verrouillable selon la revendication 7, dans laquelle l'anneau (8, 60) comprend deux ouvertures d'anneau pour suture (32) positionnées à l'opposé l'une de l'autre.

13. Élément de blocage de suture non verrouillable selon l'une quelconque des revendications 1 à 12, dans laquelle le bouchon (3, 29) a une ou plusieurs ouvertures de bouchon pour suture (38, 40, 70) s'étendant à travers le bouchon à partir de l'extrémité proximale vers l'extrémité distale du bouchon (3, 29), chaque ouverture de bouchon pour suture (40, 70) étant conçue pour recevoir une suture.

14. Élément de blocage de suture non verrouillable selon la revendication 13, dans laquelle le bouchon (3, 29) a une seule ouverture de bouchon pour suture (38) située dans le centre radial du bouchon (3, 29).

15. Élément de blocage de suture non verrouillable selon la revendication 13, dans laquelle le bouchon (3, 29) a deux ouvertures de bouchon pour suture (40, 70), situées à l'opposé l'une de l'autre et à proximité du bord radial de la tête (34, 64) du bouchon (3, 29, 62).
